# EUROPEAN PATENT APPLICATION

(11) **EP 4 800 085 A1**
(43) Date of publication of application: **02.09.2026**
(21) Application number: 25863288.4
(22) Date of filing: 01.09.2025
(51) Int. Cl.: C09K 5/04, B01D 53/04, B01D 53/14, B01D 53/26, B01D 53/28, C07C 17/389, C07C 21/18

(54) **METHOD FOR PRODUCING REGENERATED REFRIGERANT COMPOSITION CONTAINING (E)-1,2-DIFLUOROETHYLENE**

(30) Priority: 06.09.2024 JP 2024153593
(71) Applicant: Daikin Industries, Ltd., Osaka-shi, Osaka 530-0001 (JP)
(72) Inventor: SHIBATA, Kentaro, Osaka-Shi, Osaka 530-0001 (JP); KARUBE, Daisuke, Osaka-Shi, Osaka 530-0001 (JP); TSUCHIYA, Tatsumi, Osaka-Shi, Osaka 530-0001 (JP)
(74) Representative: Hoffmann Eitle
(86) International application number: PCT/JP2025/030739
(87) International publication number: WO 2026/053901

(57) **Abstract**

Provided is a method for producing a reclaimed refrigerant composition in which disproportionation of the reclaimed refrigerant composition to be obtained is suppressed, and additionally, acid generation resulting from the reaction between the refrigerant composition and oxygen is suppressed, as compared with known methods. Specifically, provided is a method for producing a reclaimed refrigerant composition comprising (E)-1,2-difluoroethylene, the method comprising step 1 of removing oxygen and water from a mixed refrigerant composition to be treated, the mixed refrigerant composition to be treated comprising the (E)-1,2-difluoroethylene, wherein step 1 is performed at a pressure of 5 MPa·G or less.

## Description

### Technical Field

The present disclosure relates to a method for producing a reclaimed refrigerant composition comprising (E)-1,2-difluoroethylene.

### Background Art

(E)-1,2-Difluoroethylene (also referred to below as "R-1132(E)"), which has a low global warming potential (GWP), is attracting attention as an alternative refrigerant for difluoromethane (R-32) or 1,1,1,2,2-pentafluoroethane (R-125), which are greenhouse gases.

PTL 1 discloses a method for reclaiming a refrigerant, the method comprising transferring an unreclaimed refrigerant composition comprising one or more hydrofluoroolefins from a source vessel to a treatment vessel, determining a target composition, determining one or more treatments based on the target composition, and performing at least one treatment selected from the group consisting of transferring, blending, distillation, nitrogen purging, filtration, dehydration, caustic scrubbing, decanting, and combinations thereof to form a partially reclaimed refrigerant composition or a reclaimed refrigerant composition.

### Citation List

### Patent Literature

PTL 1: JP2022-524758A

### Summary of Invention

### Technical Problem

An object of the present disclosure is to provide a method for producing a reclaimed refrigerant composition in which disproportionation of the reclaimed refrigerant composition to be obtained is suppressed, and additionally, acid generation resulting from the reaction between the refrigerant composition and oxygen is suppressed, as compared with known methods.

### Solution to Problem

The present disclosure encompasses, for example, the disclosures described in the following items.
1. A method for producing a reclaimed refrigerant composition comprising (E)-1,2-difluoroethylene, the method comprising:
   step 1 of removing oxygen and water from a mixed refrigerant composition to be treated, the mixed refrigerant composition to be treated comprising the (E)-1,2-difluoroethylene;
   wherein step 1 is performed at a pressure of 5 MPa·G or less.
2. The production method according to Item 1, wherein in step 1, the mixed refrigerant composition to be treated is passed through an adsorption column containing at least a dehydrating agent, the dehydrating agent being at least one selected from the group consisting of zeolite, silica gel, calcium chloride, and sodium sulfate.
3. The production method according to Item 1 or 2, wherein in step 1, oxygen is removed by passing the mixed refrigerant composition to be treated through the adsorption column further containing an oxygen scavenger and/or by using a distillation column.
4. The production method according to Item 3, wherein the oxygen scavenger is at least one selected from the group consisting of metals, metal oxides, and organic deoxidizing agents.
5. The production method according to any one of Items 1 to 4, wherein step 1 is performed at a pressure of 0.05 MPa·G or more.
6. The production method according to any one of Items 1 to 5, wherein step 1 is performed at a temperature of 150°C or lower.
7. The production method according to any one of Items 1 to 6,
   wherein the content of the (E)-1,2-difluoroethylene in the mixed refrigerant composition to be treated is more than 70 mass% and 100 mass% or less based on the total content of refrigerants in the mixed refrigerant composition to be treated taken as 100 mass%, and
   step 1 is performed at a pressure of 1.5 MPa·G or less and at a temperature of 150°C or lower.
8. The production method according to any one of Items 1 to 6,
   wherein the content of the (E)-1,2-difluoroethylene in the mixed refrigerant composition to be treated is more than 50 mass% and 70 mass% or less based on the total content of refrigerants in the mixed refrigerant composition to be treated taken as 100 mass%, and
   step 1 is performed at a pressure of 3 MPa·G or less and at a temperature of 150°C or lower.
**9.** The production method according to any one of Items 1 to 6,
   wherein the content of the (E)-1,2-difluoroethylene in the mixed refrigerant composition to be treated is 50 mass% or less based on the total content of refrigerants in the mixed refrigerant composition to be treated taken as 100 mass%, and
   step 1 is performed at a pressure of 5 MPa·G or less and at a temperature of 150°C or lower.
10. The production method according to any one of Items 1 to 9, wherein in step 1, the mixed refrigerant composition to be treated is further passed through an adsorption column containing a deacidifying agent.
11. The production method according to any one of Items 1 to 10, further comprising step 2 of capturing the reclaimed refrigerant composition.

### Advantageous Effects of Invention

According to the method for producing a reclaimed refrigerant composition of the present disclosure, disproportionation of the reclaimed refrigerant composition to be obtained is suppressed, and additionally, acid generation resulting from the reaction between the refrigerant composition and oxygen can be suppressed, as compared with known methods.

### Brief Description of Drawings

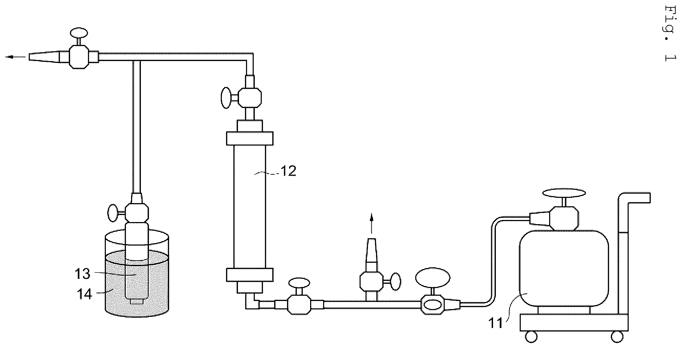

Fig. 1 is a diagram illustrating a step of the production method according to the present disclosure.

### Description of Embodiments

In the present specification, a numerical range indicated by "**. . .** to **. . .**" means a range including the numerical values before and after "to" as the lower limit and the upper limit.

In the present specification, the term "refrigerant" includes at least compounds that are specified in ISO817 (International Organization for Standardization), and that are given a refrigerant number (ASHRAE number) representing the type of refrigerant with "R" at the beginning; and further includes refrigerants that have properties equivalent to those of such refrigerants, even though a refrigerant number is not yet given. Refrigerants are broadly divided into fluorocarbon compounds and non-fluorocarbon compounds in terms of the structure of the compounds. Fluorocarbon compounds include chlorofluorocarbons (CFCs), hydrochlorofluorocarbons (HCFCs), and hydrofluorocarbons (HFCs). Non-fluorocarbon compounds include propane (R290), propylene (R1270), butane (R600), isobutane (R600a), carbon dioxide (R744), ammonia (R717), and the like.

A composition comprising a refrigerant at least includes (1) a refrigerant itself (including a mixture of refrigerants), (2) a composition that further comprises other components and that can be mixed with at least a refrigeration oil to obtain a working fluid for a refrigerating machine, and (3) a working fluid for a refrigerating machine containing a refrigeration oil. In the present specification, of these three embodiments, the composition (2) is referred to as a "refrigerant composition" so as to distinguish it from a refrigerant itself (including a mixture of refrigerants). In the production method of the present disclosure, a mixed refrigerant composition to be treated is collected from the market etc. and used as a starting material gas. The mixed refrigerant composition to be treated can comprise an oil, such as the refrigeration oil mentioned above.

The method for producing a reclaimed refrigerant composition comprising (E)-1,2-difluoroethylene according to the present disclosure comprises step 1 of removing oxygen and water from a mixed refrigerant composition to be treated, the mixed refrigerant composition to be treated comprising the (E)-1,2-difluoroethylene, wherein step 1 is performed at a pressure of 5 MPa·G or less.

The production method of the present disclosure comprises step 1 of removing oxygen and water from a mixed refrigerant composition to be treated that has been collected after use, thereby enabling the formation of a reclaimed refrigerant composition comprising (E)-1,2-difluoroethylene (R-1132(E)), and enabling the production of a reclaimed refrigerant composition comprising R-1132(E). Furthermore, since step 1 of the production method of the present disclosure is performed at a pressure of 5 MPa·G or less, disproportionation is suppressed. Additionally, the production method of the present disclosure is capable of suppressing acid generation resulting from the reaction between the refrigerant composition and oxygen.

The production method of the present disclosure enables refrigerant reclamation. In the present specification, "refrigerant reclamation" means reprocessing that involves removal of oil, water, acidic substances, and other impurities that can negatively affect the refrigerant quality, i.e., the refrigerant performance.

The reclaimed refrigerant composition produced according to the production method of the present disclosure is a refrigerant composition obtained by subjecting a used (or collected) mixed refrigerant to be treated to reclamation treatment so as to meet AHRI 700 specifications. The refrigerant quality can be verified by analytical techniques, such as GC-FID, GC-TCD, GC-MS, FTIR, Goetz Bub, Karl Fischer, Byk-Garner Color, and various other analytical methods.

### Step 1

In step 1 of the production method of the present disclosure, a mixed refrigerant composition to be treated (starting material gas) is used. The mixed refrigerant composition to be treated is collected from the market or the like. The lower limit of the content of R-1132(E) in the starting material gas is not limited and can be 0.1 mass% or more, 1 mass% or more, 10 mass% or more, 20 mass% or more, 25 mass% or more, 30 mass% or more, 40 mass% or more, 50 mass% or more, or 70 mass% or more, based on the starting material gas taken as 100 mass%. The upper limit of the content of R-1132(E) in the starting material gas is not limited and can be 100 mass% or less, 99.9 mass% or less, or 99.5 mass% or less, based on the starting material gas taken as 100 mass%.

In the production method of the present disclosure, step 1 is performed at a pressure of 5 MPa·G or less. If the pressure exceeds 5 MPa, disproportionation of the reclaimed refrigerant composition to be produced cannot be suppressed. The pressure is preferably 4 MPa or less, more preferably 3 MPa or less, even more preferably 1 MPa or less, and most preferably 0.5 MPa or less. The lower limit of the pressure is not limited and is preferably 0.01 MPa or more, more preferably 0.03 MPa or more, and even more preferably 0.05 MPa or more. By setting the lower limit of the pressure within the above ranges, gases can be prevented from entering the reaction system from the outside.

In the present specification, the pressure is gauge pressure.

In the production method of the present disclosure, step 1 is preferably performed at a temperature of 200°C or lower, more preferably 150°C or lower, and even more preferably 100°C or lower. By setting the upper limit of the temperature within the above ranges, disproportionation of the reclaimed refrigerant composition to be produced can be further suppressed. Further, step 1 is preferably performed at a temperature of -53°C or higher, more preferably 0°C or higher, and even more preferably higher than 0°C. Setting the lower limit of the temperature within the above ranges is desirable because the mixed refrigerant composition to be treated is in a liquid or gaseous state, and thus step 1 can be more easily performed. By setting the upper limit of the temperature within the above ranges, disproportionation can be further suppressed.

In the production method of the present disclosure, suitable pressure and temperature conditions in step 1 can also be set according to the range of the content of R-1132(E) in the mixed refrigerant composition to be treated. The following description is provided based on the range of the content of R-1132(E) in the mixed refrigerant composition to be treated. In cases (1) to (3) below, the "content of (E)-1,2-difluoroethylene" refers to a content based on the total content of refrigerants in the mixed refrigerant composition to be treated taken as 100 mass%. The "refrigerants" here refer to, for example, R-1132(E), R-1234yf, and the like, and are based on the concept of not including impurities, such as oil, contained in the mixed refrigerant composition to be treated.

### (1) Content of R-1132(E): More than 70 Mass% and 100 Mass% or Less

When the content of R-1132(E) in the mixed refrigerant composition to be treated is more than 70 mass% and 100 mass% or less based on the total content of refrigerants in the mixed refrigerant composition to be treated taken as 100 mass%, the pressure in step 1 is preferably 1.5 MPa·G or less, more preferably 1 MPa·G or less, and even more preferably 0.5 MPa·G or less. In this case, the lower limit of the pressure in step 1 is not limited and may be 0.01 MPa or more, 0.03 MPa or more, or 0.05 MPa or more. When the content of R-1132(E) in the mixed refrigerant composition to be treated is more than 70 mass% and 100 mass% or less based on the total content of refrigerants in the mixed refrigerant composition to be treated taken as 100 mass%, setting the pressure in step 1 within the above ranges further suppresses disproportionation of the reclaimed refrigerant composition to be obtained and also further suppresses acid generation resulting from the reaction between the refrigerant composition and oxygen.

When the content of R-1132(E) in the mixed refrigerant composition to be treated is more than 70 mass% and 100 mass% or less based on the total content of refrigerants in the mixed refrigerant composition to be treated taken as 100 mass%, the temperature in step 1 is preferably 150°C or lower, more preferably 100°C or lower, and even more preferably 50°C or lower. In this case, the upper limit of the temperature in step 1 is not limited and may be about 200°C or lower. Further, in this case, the lower limit of the temperature in step 1 is not limited and may be -79°C or higher, -30°C or higher, 0°C or higher, or 20°C or higher. When the content of R-1132(E) in the mixed refrigerant composition to be treated is more than 70 mass% and 100 mass% or less based on the total content of refrigerants in the mixed refrigerant composition to be treated taken as 100 mass%, setting the temperature in step 1 within the above ranges further suppresses disproportionation of the reclaimed refrigerant composition to be obtained and also further suppresses acid generation resulting from the reaction between the refrigerant composition and oxygen.

### (2) Content of R-1132(E): More than 50 Mass% and 70 Mass% or Less

When the content of R-1132(E) in the mixed refrigerant composition to be treated is more than 50 mass% and 70 mass% or less based on the total content of refrigerants in the mixed refrigerant composition to be treated taken as 100 mass%, the pressure in step 1 is preferably 3 MPa·G or less, and more preferably 2 MPa·G or less. In this case, the lower limit of the pressure in step 1 is not limited and may be 0.01 MPa or more, 0.03 MPa or more, or 0.05 MPa or more. When the content of R-1132(E) in the mixed refrigerant composition to be treated is more than 50 mass% and 70 mass% or less based on the total content of refrigerants in the mixed refrigerant composition to be treated taken as 100 mass%, setting the pressure in step 1 within the above ranges further suppresses disproportionation of the reclaimed refrigerant composition to be obtained and also further suppresses acid generation resulting from the reaction between the refrigerant composition and oxygen.

When the content of R-1132(E) in the mixed refrigerant composition to be treated is more than 50 mass% and 70 mass% or less based on the total content of refrigerants in the mixed refrigerant composition to be treated taken as 100 mass%, the temperature in step 1 is preferably 150°C or lower, and more preferably 100°C or lower. In this case, the upper limit of the temperature in step 1 is not limited and may be about 200°C or lower. Further, in this case, the lower limit of the temperature in step 1 is not limited and may be -79°C or higher, -30°C or higher, 0°C or higher, or 20°C or higher. When the content of R-1132(E) in the mixed refrigerant composition to be treated is more than 50 mass% and 70 mass% or less based on the total content of refrigerants in the mixed refrigerant composition to be treated taken as 100 mass%, setting the temperature in step 1 within the above ranges further suppresses disproportionation of the reclaimed refrigerant composition to be obtained and also further suppresses acid generation resulting from the reaction between the refrigerant composition and oxygen.

### (3) Content of R-1132(E): 50 Mass% or Less

When the content of R-1132(E) in the mixed refrigerant composition to be treated is 50 mass% or less based on the total content of refrigerants in the mixed refrigerant composition to be treated taken as 100 mass%, the pressure in step 1 is preferably 5 MPa·G or less, and more preferably 4 MPa·G or less. In this case, the lower limit of the pressure in step 1 is not limited and may be 0.01 MPa or more, 0.03 MPa or more, or 0.05 MPa or more. When the content of R-1132(E) in the mixed refrigerant composition to be treated is 50 mass% or less based on the total content of refrigerants in the mixed refrigerant composition to be treated taken as 100 mass%, setting the pressure in step 1 within the above ranges further suppresses disproportionation of the reclaimed refrigerant composition to be obtained and also further suppresses acid generation resulting from the reaction between the refrigerant composition and oxygen.

When the content of R-1132(E) in the mixed refrigerant composition to be treated is 50 mass% or less based on the total content of refrigerants in the mixed refrigerant composition to be treated taken as 100 mass%, the temperature in step 1 is preferably 150°C or lower. In this case, the upper limit of the temperature in step 1 is not limited and may be about 200°C or lower. Further, in this case, the lower limit of the temperature in step 1 is not limited and may be -79°C or higher, -30°C or higher, 0°C or higher, or 20°C or higher. When the content of R-1132(E) in the mixed refrigerant composition to be treated is 50 mass% or less based on the total content of refrigerants in the mixed refrigerant composition to be treated taken as 100 mass%, setting the temperature in step 1 within the above ranges further suppresses disproportionation of the reclaimed refrigerant composition to be obtained and also further suppresses acid generation resulting from the reaction between the refrigerant composition and oxygen.

In step 1, oxygen and water are removed from the mixed refrigerant composition to be treated. In step 1, oxygen is preferably removed by passing the mixed refrigerant composition to be treated through an adsorption column containing at least a dehydrating agent.

The dehydrating agent is not limited and may be selected from zeolite, silica gel, calcium chloride, sodium sulfate, and the like. Among these, zeolite is preferred due to its greater dehydrating ability.

The dehydrating agent may be used alone or as a mixture of two or more.

When the volume of the adsorption column is about 50 to 100 cc, the amount of the dehydrating agent used in the adsorption column is preferably 10 to 60 g, and more preferably 20 to 50 g. By setting the lower limit of the amount of the dehydrating agent used within the above ranges, the reclaimed refrigerant composition is more sufficiently dehydrated. By setting the upper limit of the amount of the dehydrating agent used within the above ranges, the gas flow inside the reaction device is improved, and production efficiency is further enhanced.

In the present specification, when the adsorption column contains only the dehydrating agent and does not contain the oxygen scavenger, deacidifying agent, or the like described below, the adsorption column is also referred to as a "dehydration column."

The method for oxygen removal from the mixed refrigerant composition to be treated is not limited. Examples include a method comprising passing the mixed refrigerant composition to be treated through an adsorption column containing the dehydrating agent described above and further containing an oxygen scavenger, a method comprising using the difference between the boiling point of the mixed refrigerant composition to be treated and the boiling point of oxygen to remove oxygen, and the like. Examples of the method comprising using the difference between the boiling point of the mixed refrigerant composition to be treated and the boiling point of oxygen to remove oxygen include a method for oxygen removal by using a distillation column, a method comprising cooling the mixed refrigerant composition to be treated to a liquid or solid state and removing oxygen in its gaseous state, and the like. These methods may be used singly or in a combination of two or more.

The oxygen scavenger is not limited and may be selected from metals, metal oxides, metal salts, organic deoxidizing agents, sulfates, and the like.

Examples of the metals include iron, and iron powder can be suitably used.

Examples of the metal oxides include metal oxides other than those used as dehydrating agents. Specifically, triiron tetraoxide and the like may be used.

Examples of the organic deoxidizing agents include vitamin C.

When the volume of the adsorption column is about 50 to 100 cc, the amount of the oxygen scavenger used in the adsorption column is preferably 10 to 60 g, and more preferably 20 to 50 g. By setting the lower limit of the amount of the oxygen scavenger used within the above ranges, oxygen is more sufficiently removed from the reclaimed refrigerant composition. By setting the upper limit of the amount of the oxygen scavenger used within the above ranges, the gas flow inside the reaction device is improved, and production efficiency is further enhanced.

When the adsorption column is used in the production method of the present disclosure, the adsorption column may contain other additives, such as polymerization inhibitors and stabilizers, in addition to the dehydrating agent and the oxygen scavenger described above. When the adsorption column is used in the production method of the present disclosure, a single adsorption column may contain the dehydrating agent, the oxygen scavenger, and other additives, such as polymerization inhibitors and stabilizers. Alternatively, multiple adsorption columns each containing those substances separately may be connected in series. Alternatively, multiple adsorption columns containing different combinations of the dehydrating agent, the oxygen scavenger, and other additives, such as polymerization inhibitors and stabilizers, may be connected in series. The other additives, such as polymerization inhibitors and stabilizers, may be used by being contained in an adsorption column or by being added to the mixed refrigerant composition to be treated.

The polymerization inhibitor for use may be limonene, terpenes, and the like. The addition of the polymerization inhibitor to the adsorption column enables regeneration of the dehydrating agent and the oxygen scavenger. The polymerization inhibitor may be used by being contained in the adsorption column or by being added to the mixed refrigerant composition to be treated. When the polymerization inhibitor is used as described above, the polymerization inhibitor eluted from the adsorption column into the mixed refrigerant composition to be treated, or the polymerization inhibitor added to the mixed refrigerant composition to be treated, will be mixed into the mixed refrigerant composition to be treated. However, by connecting an adsorption column containing silica gel, the polymerization inhibitor that has mixed into the mixed refrigerant composition to be treated can be removed. The adsorption column containing silica gel is preferably connected downstream of the adsorption column containing the polymerization inhibitor. The polymerization inhibitor may be used by being contained in the adsorption column or by being added to the mixed refrigerant composition to be treated.

The stabilizer for use may be dibutylhydroxytoluene (BHT), butylated hydroxyanisole (BHA), and the like. When the mixed refrigerant composition to be treated passes through the adsorption column, R-1132(E) may undergo polymerization or decomposition due to the effects of the dehydrating agent, the oxygen scavenger, and the other additives. The addition of the stabilizer to the adsorption column enables suppression of the polymerization or decomposition of R-1132(E). The stabilizer may be used by being contained in the adsorption column or by being added to the mixed refrigerant composition to be treated.

In the production method of the present disclosure, oxygen may be removed from the mixed refrigerant composition to be treated by using a distillation column. The distillation column is not limited, and any known distillation column may be used.

For removing oxygen using the distillation column, the distillation temperature is preferably 50°C to 250°C, more preferably 80°C to 200°C, even more preferably 100°C to 180°C, and most preferably 130°C to 170°C.

In the production method of the present disclosure, the adsorption column may further contain a deacidifying agent. When a deacidifying agent is contained in the adsorption column, acid components generated by the reaction between R-1132(E) in the mixed refrigerant composition to be treated and oxygen mixed into the mixed refrigerant composition to be treated from the air can be removed at the same time that water is removed using the adsorption column.

The deacidifying agent is not limited and can be selected from soda lime, sodium hydroxide, potassium hydroxide, and the like. Among these, soda lime is preferred from the viewpoint of more easily removing acid components.

When the volume of the adsorption column is about 50 to 100 cc, the amount of the deacidifying agent used in the adsorption column is preferably 10 to 60 g, and more preferably 20 to 50 g. By setting the lower limit of the amount of the deacidifying agent used within the above ranges, the acid components in the reclaimed refrigerant composition are more sufficiently removed. By setting the upper limit of the amount of the deacidifying agent used within the above ranges, the gas flow inside the reaction device is improved, and production efficiency is further enhanced.

The deacidifying agent may be contained in the adsorption column containing the dehydrating agent and the oxygen scavenger. Alternatively, an additional adsorption column may be incorporated into the reaction system, and the deacidifying agent may be contained in the additional adsorption column.

When the deacidifying agent is contained in an adsorption column separate from the adsorption column containing the dehydrating agent and the oxygen scavenger, the adsorption column containing the deacidifying agent is preferably located upstream of the adsorption column containing the dehydrating agent and the oxygen scavenger. In this configuration, the deacidifying agent will be trapped in the adsorption column containing the dehydrating agent and the oxygen scavenger, thereby enabling the removal of the deacidifying agent from the reclaimed refrigerant composition to be produced.

Step 1 described above enables the production of a reclaimed refrigerant composition comprising (E)-1,2-difluoroethylene.

### Cooling Step

Before step 2 described below, step 1 may comprise a cooling step of cooling the obtained reclaimed refrigerant composition to obtain a liquefied or solidified reclaimed refrigerant composition. When step 1 comprises the cooling step, the produced reclaimed refrigerant composition can be more efficiently collected.

The method for cooling the reclaimed refrigerant composition is not limited. For example, as shown in Fig. 1, the mixed refrigerant composition to be treated is passed through an adsorption column 12 containing a dehydrating agent and an oxygen scavenger to produce a reclaimed refrigerant composition, and the produced reclaimed refrigerant composition may be cooled by collecting it in a cylinder 13 immersed in a cooling vessel filled with liquid nitrogen 14 as a coolant.

The lower limit of the cooling temperature in the cooling step is preferably -230°C or higher, and more preferably -210°C or higher. The upper limit of the cooling temperature in the cooling step is preferably 50°C or lower, 25°C or lower, 0°C or lower, -50°C or lower, -100°C or lower, -150°C or lower, or -170°C or lower. By setting the cooling temperature within the above ranges, the reclaimed refrigerant composition produced according to the production method of the present disclosure can be collected more efficiently.

In the production method of the present disclosure, when step 1 comprises the cooling step, oxygen may also be removed by performing a degassing operation in the cylinder used in the cooling step. That is, as shown in Fig. 1, the reclaimed refrigerant composition is collected in the cylinder 13 immersed in the cooling vessel filled with liquid nitrogen 14 as a coolant, and when doing so, a degassing operation may be performed in the cylinder 13.

Step 1 described above enables the removal of oxygen and water from the mixed refrigerant composition to be treated, the mixed refrigerant composition to be treated comprising (E)-1,2-difluoroethylene.

### Step 2

After step 1 described above, the production method of the present disclosure may further comprise step 2 of capturing the reclaimed refrigerant composition. Step 2 in the production method of the present disclosure enables capturing the produced reclaimed refrigerant composition in a capture vessel with a larger volume.

The method for capturing the reclaimed refrigerant composition is not limited. By connecting a vessel containing the reclaimed refrigerant composition obtained in step 1 to a capture vessel with a larger volume and reducing the pressure, the reclaimed refrigerant composition can be captured in the capture vessel.

In step 2, the capture vessel is preferably cooled by immersion in an ice bath. The cooling temperature is preferably -10°C to 10°C, and more preferably -5°C to 5°C. Setting the cooling temperature within the above ranges enables more stably capturing the reclaimed refrigerant composition in the capture vessel.

The production method of the present disclosure described above enables the reclamation of a mixed refrigerant composition to be treated that has been collected from the market, and enables the production of a reclaimed refrigerant composition comprising (E)-1,2-difluoroethylene. The reclaimed refrigerant composition produced according to the production method of the present disclosure has a low global warming potential (GWP) and can be suitably used as an alternative refrigerant to difluoromethane (R-32) or 1,1,1,2,2-pentafluoroethane (R-125), which are greenhouse gases. The production method of the present disclosure suppresses disproportionation of the reclaimed refrigerant composition to be produced, and additionally, enables the suppression of acid generation resulting from the reaction between the refrigerant composition and oxygen, thus enabling appropriate production of a reclaimed refrigerant composition comprising (E)-1,2-difluoroethylene.

### Examples

The present disclosure is described in more detail below with reference to Examples and Comparative Examples. However, the present disclosure is not limited to these Examples.

### Example 1

A reclaimed refrigerant composition comprising (E)-1,2-difluoroethylene was produced by using a production device similar to that shown in Fig. 1. Specifically, a vessel 11 containing a mixed refrigerant composition to be treated that was collected from the market was prepared and connected to the production device. Subsequently, the mixed refrigerant composition to be treated in the gas phase was transferred to the production device and passed through an adsorption column 12 containing a deacidifying agent and a dehydrating agent to remove water and acids. The resulting mixed refrigerant composition to be treated was collected in a cylinder 13, which was immersed in a cooling vessel filled with liquid nitrogen 14 as a coolant. Subsequently, a degassing operation was performed in the cylinder 13 to remove oxygen from the mixed refrigerant composition to be treated, thereby producing a reclaimed refrigerant composition.

The above production of the reclaimed refrigerant composition comprising (E)-1,2-difluoroethylene was performed under the following conditions. Fig. 1 shows an example in which a single adsorption column 12 is provided; however, in Example 1, the mixed refrigerant composition to be treated was first passed through a deacidification column from the supply side of the mixed refrigerant composition to be treated and then through a dehydration column connected in series to the deacidification column.
- Pressure in the reaction system: 0.05 to 0.1 MPa·G
- Temperature in the reaction system: 23°C
- Degassing operation in the cylinder: 1 time/100 g
- Deacidification column: volume: 68 cc
- Deacidifying agent: soda lime, filling amount: 43 g
- Flow rate through the deacidification column: 5 g/min
- Dehydration column: volume: 68 cc, MS dehydrating agent (a molecular sieve (zeolite)), filling amount: 43 g
- MS regeneration conditions: 150°C under reduced pressure, regeneration time: 3 hours or more
- Flow rate through the dehydration column: 5 g/min; moisture content reduced from 282 ppm to 8 ppm

### Example 2

A reclaimed refrigerant composition comprising (E)-1,2-difluoroethylene was produced in the same manner as in Example 1, except for the following points. Specifically, the mixed refrigerant composition to be treated in the gas phase was transferred to the production device and passed through an adsorption column (dehydration column) 12 containing a dehydrating agent and not containing an oxygen scavenger. After the adsorption column (dehydration column) 12, the mixed refrigerant composition to be treated was passed through a distillation column for deoxygenation.

The above production of the reclaimed refrigerant composition comprising (E)-1,2-difluoroethylene using the distillation column was performed under the following conditions.
- Flow rate through the dehydration column: 80 g/min
- Pressure in the dehydration column: 0.4 to 0.6 MPa·G
- Temperature in the dehydration column: 2 to 6°C
- Distillation temperature: 35°C

### Evaluation Methods

The reclaimed refrigerant compositions obtained in the Examples were evaluated as described below.

### Composition of Reclaimed Refrigerant Compositions

The composition of each of the reclaimed refrigerant compositions was analyzed using gas chromatography (MS detector).

### Moisture Content

The moisture content of each of the reclaimed refrigerant compositions was measured using a Karl Fischer moisture meter.

### Acidity

The acidity of each of the reclaimed refrigerant compositions was measured by immersing pH test paper into it.

### Oxygen Concentration

The oxygen concentration of each of the reclaimed refrigerant compositions was measured using an oxygen concentration meter.

### Evaporation Residue Content

The content of evaporation residue, such as refrigeration oil, in each of the reclaimed refrigerant compositions was measured in accordance with the AHRI 700 specifications.

The results are shown in Table 1.

**Table 1**

| | | Example 1 | | Example 2 | |
|---|---|---|---|---|---|
| Pressure (MPa·G) | | 0.05 to 0.1 | | 0.05 to 0.1 | |
| Composition | | Mixed refrigerant composition to be treated (before reclamation) | Reclaimed refrigerant composition (after reclamation) | Mixed refrigerant composition to be treated (before reclamation) | Reclaimed refrigerant composition (after reclamation) |
| Composition of refrigerant | R-1132(E) | 26.8 mass% | 28.8 mass% | 100 mass% | 100 mass% |
| | R-1234yf | 73.2 mass% | 71.2 mass% | 0 mass% | 0 mass% |
| Impurities | Moisture content | 282 ppm by mass (gas phase) | 8 ppm by mass (gas phase) | 157 ppm by mass (liquid phase) | 6 ppm by mass (liquid phase) |
| | Acidity (analysis of the gas phase) | Detected | Not detected | Detected | Not detected |
| | Evaporation residue content (analysis of the liquid phase) | 3.8 mass% | < 0.001 mass% | **<** 0.001 mass% | < 0.001 mass% |
| | Oxygen concentration (analysis of the gas phase) | 30,000 ppm by volume or less | 500 ppm by volume or less | 350 ppm by volume | 11 ppm by volume |

The results shown in Table 1 reveal the following. In Examples 1 and 2, the moisture content, evaporation residue content (e.g., refrigeration oil), acidity, and oxygen content of the reclaimed refrigerant compositions after reclamation were lower than those of the mixed refrigerant composition to be treated before reclamation, demonstrating successful production of reclaimed refrigerant compositions.

The results shown in Table 1 also reveal that disproportionation was suppressed in Examples 1 and 2. The results also reveal that in Example 1, acid generation due to the reaction between the refrigerant composition and oxygen was suppressed, and that in Example 2, oxygen was removed and acid generation was suppressed.

### Description of Reference Numerals

11: Vessel containing a mixed refrigerant composition to be treated
12: Adsorption column
13: Cylinder
14: Liquid nitrogen

## Claims

1. A method for producing a reclaimed refrigerant composition comprising (E)-1,2-difluoroethylene, the method comprising:
step 1 of removing oxygen and water from a mixed refrigerant composition to be treated, the mixed refrigerant composition to be treated comprising the (E)-1,2-difluoroethylene;
wherein step 1 is performed at a pressure of 5 MPa·G or less.

2. The production method according to claim 1, wherein in step 1, the mixed refrigerant composition to be treated is passed through an adsorption column containing at least a dehydrating agent, the dehydrating agent being at least one selected from the group consisting of zeolite, silica gel, calcium chloride, and sodium sulfate.

3. The production method according to claim 2, wherein in step 1, oxygen is removed by passing the mixed refrigerant composition to be treated through the adsorption column further containing an oxygen scavenger and/or by using a distillation column.

4. The production method according to claim 3, wherein the oxygen scavenger is at least one selected from the group consisting of metals, metal oxides, and organic deoxidizing agents.

5. The production method according to claim 1, wherein step 1 is performed at a pressure of 0.05 MPa·G or more.

6. The production method according to claim 1, wherein step 1 is performed at a temperature of 150°C or lower.

7. The production method according to claim 1,
wherein the content of the (E)-1,2-difluoroethylene in the mixed refrigerant composition to be treated is more than 70 mass% and 100 mass% or less based on the total content of refrigerants in the mixed refrigerant composition to be treated taken as 100 mass%, and
step 1 is performed at a pressure of 1.5 MPa·G or less and at a temperature of 150°C or lower.

8. The production method according to claim 1,
wherein the content of the (E)-1,2-difluoroethylene in the mixed refrigerant composition to be treated is more than 50 mass% and 70 mass% or less based on the total content of refrigerants in the mixed refrigerant composition to be treated taken as 100 mass%, and
step 1 is performed at a pressure of 3 MPa·G or less and at a temperature of 150°C or lower.

9. The production method according to claim 1,
wherein the content of the (E)-1,2-difluoroethylene in the mixed refrigerant composition to be treated is 50 mass% or less based on the total content of refrigerants in the mixed refrigerant composition to be treated taken as 100 mass%, and
step 1 is performed at a pressure of 5 MPa·G or less and at a temperature of 150°C or lower.

10. The production method according to claim 1, wherein in step 1, the mixed refrigerant composition to be treated is further passed through an adsorption column containing a deacidifying agent.

11. The production method according to claim 1, further comprising step 2 of capturing the reclaimed refrigerant composition.
